# EUROPEAN PATENT APPLICATION

(11) **EP 4 570 780 A1**
(43) Date of publication of application: **18.06.2025**
(21) Application number: 23872745.7
(22) Date of filing: 25.07.2023
(51) Int. Cl.: C07C 2/74, C07C 13/48, C07C 15/08, C07C 15/24

(54) **PROCESS FOR REMOVING RESIDUAL HYDROGEN IN AROMATIC FRACTION**

(30) Priority: 30.09.2022 KR 20220124834
(71) Applicant: SK Innovation Co., Ltd., Seoul 03188 (KR); SK Geo Centric Co., Ltd., Jongno-gu Seoul 03161 (KR)
(72) Inventor: LEE, Sang Il, Daejeon 34124 (KR); LEE, Ji Hoon, Daejeon 34124 (KR)
(74) Representative: Frick, Robert
(86) International application number: PCT/KR2023/010709
(87) International publication number: WO 2024/071620

(57) **Abstract**

Disclosed is a process of removing residual hydrogen from an aromatic fraction having a reduced bromine index through selective hydrogenation by reaction with olefins and/or bicyclic-aromatic components contained therein.

## Description

### [Technical Field]

The present disclosure relates to a process of removing residual hydrogen in aromatic fractions. More particularly, the present disclosure relates to a process of removing residual hydrogen in an aromatic fraction by reaction with olefins and/or bicyclic-aromatic components contained therein, where the aromatic fraction has already been subject to reduction in bromine index by preliminary (or prior) selective hydrogenation.

### [Background Art]

C6+ aromatic hydrocarbons, particularly benzene, toluene, or mixed xylenes (or xylene isomers), account for a large portion of sources for basic chemicals used in the petrochemical field. Among them, mixed xylenes including meta-xylene (m-xylene), para-xylene (p-xylene), and ortho-xylene (o-xylene), particularly, p-xylene, are highly essential.

Commercial methods of preparing benzene, toluene or mixed xylene include separation/recovery methods from aromatic-rich fractions and synthesis methods. As for the separation/recovery methods, mixed xylenes are separated through distillation of reformates obtained by catalytic reforming of naphtha, or separated from pyrolysis oil generated as a byproduct upon thermal cracking of naphtha.

However, aromatic hydrocarbon fractions such as catalytic reformates as well as pyrolysis oils contain unsaturated hydrocarbons (e.g., olefin, acetylene, and/or styrene or derivatives thereof), which have undesirable effects on operation in the downstream processes. Therefore, techniques for reducing the content of olefins in the feedstock of the downstream process by clay pre-treatment or pre-hydrogenation are known. Among them, hydrogenation is performed by feeding hydrogen in the presence of a catalyst to convert unsaturated hydrocarbons (i.e., olefins, acetylene and/or styrene (including derivatives thereof, but excluding aromatic (benzene) rings) in aromatic fractions into saturated hydrocarbons (i.e., paraffins), but is performed to minimize aromatic loss, thus relieving problems associated with frequent replacement cycles, increase in clay pre-treatment cost and waste generation encountered in the clay treatment. In this case, a hydrogenation catalyst known in the art, for example, a nickel-based catalyst, a noble metal-based catalyst (Pt, Pd, or the like), cobalt-molybdenum (Co-Mo), nickel-molybdenum (Ni-Mo), or the like is used.

However, due to the nature of the hydrogenation reaction, aromatic fractions may be lost due to excessive hydrogenation, or all of the fed hydrogen may not be consumed and a small amount thereof may remain in hydrogenated aromatic fractions. In particular, residual hydrogen may adversely affect distillation performance in the downstream xylene process, causing cavitation due to bubbles in the pump and thus damage to the pump, or deterioration in separation performance in the para-xylene purification process.

When a catalyst having controlled hydrogenation activity, such as a NiMo sulfide catalyst, is used in order to selectively remove unsaturated hydrocarbons such as olefins by hydrogenation while suppressing the loss of aromatic fractions, a hydrogenation product initially containing residual hydrogen is discharged. On the other hand, a catalyst having a relatively high hydrogenation activity, for example, a Ni or NiMo reduction-type catalyst has almost no residual hydrogen, but causes a serious loss of aromatic fractions.

In this regard, in an attempt to minimize such aromatic loss, a method of combining a NiMo sulfide catalyst with a NiMo reduced catalyst may be considered. However, initially, almost no residual hydrogen is contained in the product, but as the reaction progresses for a predetermined time or longer, hydrogenation activity decreases and a product containing residual hydrogen is thus discharged.

In this regard, a method of removing residual hydrogen from the selective hydrogenation product using a physical method such as gas-liquid separation (e.g., a flash column, a deheptanizer, a fractionator, or the like) is also known (in Korean Patent No. 1109814, Korean Patent Laid-open Publication No. 2005-0089010, US Patent No. 6977317, or the like). However, the use of an additional separation and recovery facility for the process inevitably increases the equipment investment costs.

Meanwhile, the present applicant has developed a two-stage selective hydrogenation process based on a catalyst system in which two types of catalysts (first hydrogenation catalyst and second hydrogenation catalyst) having different hydrogenation activities are charged in a stage loading manner or in two reactors connected in series (US Patent Laid-open Publication No. 2022-0073440). The process may be performed by removing residual hydrogen in the finally discharged hydrogenated aromatic fraction in consideration of the second hydrogenation reaction conditions. However, even if the second hydrogenation reaction is delicately controlled, the content of residual hydrogen in the final hydrogenated aromatic fraction is approximately 84 wtppm (concentration of residual hydrogen in the final product after operation for 17 days) and then gradually increases. Moreover, when the conditions of the second hydrogenation are adjusted to minimize the amount of residual hydrogen in the final aromatic fraction, increased loss of monocyclic aromatics due to excessive hydrogenation is still unresolved.

As described above, even if an aromatic fraction containing a reduced amount of bromine index-increasing components (such as olefins) while minimizing aromatic loss through primary selective hydrogenation is prepared, the unreacted residual (excess) hydrogen is still a technical problem.

Therefore, there is a need for a method of effectively removing residual hydrogen present in aromatic fractions, particularly C8+ aromatic fractions without causing loss of monocyclic aromatics after selective hydrogenation.

### [Disclosure]

### [Technical Problem]

An embodiment of the present disclosure provides a method of reducing a loss of a monocyclic aromatic while effectively removing residual hydrogen in aromatic fractions having a reduced bromine index through selective hydrogenation.

### [Technical Solution]

In accordance with an aspect of the present disclosure, a process of purifying an aromatic hydrocarbon comprises:
a) performing selective hydrogenation of an aromatic hydrocarbon-containing feedstock having a bromine index of at least 30 in the presence of a selective hydrogenation catalyst while supplying hydrogen to form a first aromatic hydrocarbon-containing product having a reduced bromine index and unsaturated hydrocarbon content and containing residual hydrogen at up to 200 wtppm, and
b) performing hydrogenation of the first aromatic hydrocarbon-containing product using the residual hydrogen in the presence of a hydrogenation catalyst to form a second aromatic hydrocarbon-containing product containing residual hydrogen in a reduced amount, compared to the first aromatic hydrocarbon-containing product,
wherein the first aromatic hydrocarbon-containing product has a bromine index reduced by at least 20% compared to the aromatic hydrocarbon-containing feedstock, and contains (i) an unsaturated hydrocarbon and (ii) a bicyclic aromatic hydrocarbon.

According to an exemplary embodiment, the total aromatic loss in the first aromatic hydrocarbon-containing product is less than 0.45% by weight based on the weight of the aromatic hydrocarbon-containing feedstock, and monocyclic aromatic loss in the second aromatic hydrocarbon-containing product is less than 0.15%, based on the first aromatic hydrocarbon-containing product.

According to an exemplary embodiment, the hydrogenation of step b) may be performed under reaction conditions controlled in a temperature range higher than 55°C and lower than about 255°C, and a pressure range of 3 to 60 bar.

According to an exemplary embodiment, the hydrogenation catalyst in step b) may comprise at least one active metal selected from nickel and platinum group metals and an inorganic oxide support.

According to an exemplary embodiment, the platinum group metal may comprise at least one selected from the group consisting of platinum, rhodium, and ruthenium.

According to an exemplary embodiment, the content of the active metal in the hydrogenation catalyst in step b) may be controlled within the range of 0.1 to 40% by weight, on an elemental basis.

According to an exemplary embodiment, the aromatic hydrocarbon-containing feedstock may comprise C8+ aromatic hydrocarbons.

According to an exemplary embodiment, the method may further comprise:
c) separating the second aromatic hydrocarbon-containing product into a C9+ aromatic hydrocarbon fraction and a C8 aromatic hydrocarbon fraction, and
d) separating and recovering para-xylene from the separated C8 aromatic hydrocarbon fraction.

According to an exemplary embodiment, the method may further comprise, prior to step c), treating the second aromatic hydrocarbon-containing product with a solid acid.

According to an exemplary embodiment, the method may further comprise:
e) isomerizing the residual C8 aromatic hydrocarbon fraction not recovered as para-xylene to form a C8 aromatic hydrocarbon fraction containing an increased content of para-xylene, followed by recycling to step (c).

### [Advantageous effects]

According to an embodiment of the present disclosure, when additional hydrogenation is performed on a selective hydrogenation product having a reduced bromine index (BI) through selective hydrogenation of an aromatic hydrocarbon fraction, loss of aromatics, especially monocyclic aromatic hydrocarbons such as C8, can be minimized while effectively removing residual hydrogen in the selective hydrogenation product by controlling reaction conditions, selection of catalysts, and the content of the bicyclic-aromatic component. As such, by removing residual hydrogen in the selectively hydrogenated aromatic hydrocarbon fraction, it is possible to prevent various problems that may occur in downstream processes such as pump-based transfer, separation of xylene, and purification of para-xylene.

### [Description of Drawings]

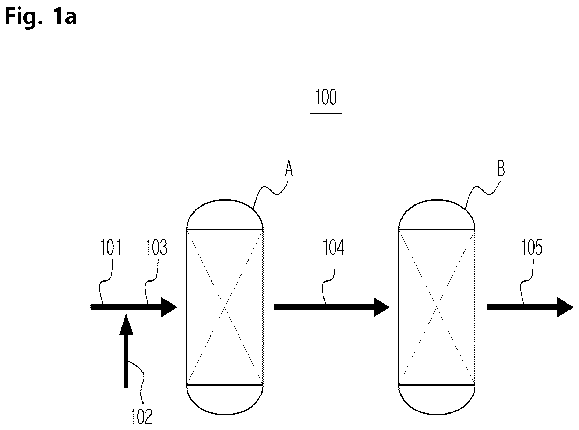

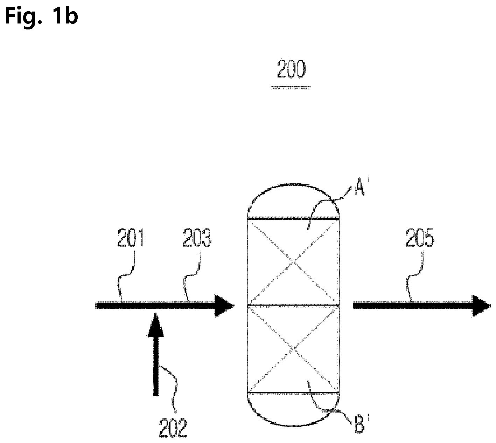

FIGS. 1A and 1B schematically illustrate an exemplary process of treating an aromatic hydrocarbon-containing feedstock in the order of selective hydrogenation and hydrogenation, and then removing residual hydrogen from the selectively hydrogenated aromatic hydrocarbon-containing product by the hydrogenation.

### [Best Mode]

The present disclosure can be implemented in its entirety with reference to the following description. It is to be understood that the following description illustrates preferable embodiments of the present disclosure, but the present disclosure is not necessarily limited thereto. It is also to be understood that the accompanying drawings are included to provide a further understanding of the present disclosure, and are not intended to limit the scope of the present disclosure.

The terms used herein are defined as follows.

As used herein, the term "heterogeneous catalyst" refers to a catalyst that is present in a different phase from a reactant in a catalytic reaction. For example, a heterogeneous catalyst may remain undissolved in a reaction medium. When a heterogeneous catalyst is used, the reaction begins with the diffusion and adsorption of reactants onto the surface of the heterogeneous catalyst. After completion of the reaction, the product needs to be desorbed from the surface of the heterogeneous catalyst.

As used herein, the term "support" refers to a material (typically a solid-phase material) having a large specific surface area, to which a catalytically active component is attached, and the support may or may not be involved in a catalytic reaction.

As used herein, the term "unsaturated hydrocarbon" means a hydrocarbon containing double bonds or even triple bonds and is typically intended to include olefins and alkynes, more specifically olefins. However, unless otherwise mentioned herein, it is understood that the aromatic ring is excluded from the range of unsaturated hydrocarbons removed by "selective hydrogenation" even though it contains a double bond. However, a bicyclic-aromatic ring falls within the range of the compound removed by "hydrogenation", like unsaturated hydrocarbons.

As used herein, the term "olefin" may be intended to include alkenes, cycloalkenes, alkenyl benzenes and the like.

As used herein, the term "Cn+ aromatic" refers to an aromatic hydrocarbon having Cn or more carbon atoms, and similarly, the term "Cn- aromatic" refers to an aromatic hydrocarbon having Cn or fewer carbon atoms.

As used herein, the term "Cn+ hydrocarbon" refers to a hydrocarbon having Cn or more carbon atoms, and similarly, the term "Cn- hydrocarbon" refers to a hydrocarbon having Cn or fewer carbon atoms.

As used herein, the term "C8 aromatic" refers to an aromatic hydrocarbon including mixed xylene (ortho-xylene, meta-xylene and para-xylene) and/or ethylbenzene.

As used herein, the term "hydrogenation" generally refers to a reaction of an organic compound with hydrogen wherein the reaction is typically performed in the presence of a catalyst. Meanwhile, in a narrow sense, the term "selective hydrogenation" means preferentially converting unsaturated hydrocarbons through hydrogenation over aromatic compound(s) in a hydrogenated feedstock. Here, "hydrogenation" is distinguished from "selective hydrogenation". Specifically, hydrogenation minimizes the saturation of the monocyclic aromatic ring, but allows saturation of the aromatic ring in the bicyclic aromatics having higher hydrogenation reactivity than the monocyclic aromatics, whereas selective hydrogenation minimizes saturation of the aromatic ring itself.

As used herein, the term "bromine index (BI)" refers to a measured value (mg) of bromine consumed by 100 g of a hydrocarbon or hydrocarbon mixture, and may be used to indicate the percentage of unsaturated bonds present in the hydrocarbon. The bromine index may be measured, for example, according to ASTM D 2710-92.

As used herein, the term "rich" means that a particular compound is present, for example, in an amount of at least about 50%, specifically at least 70%, more specifically at least about 80%, particularly specifically, at least about 90%, on a predetermined basis (e.g., on a weight, volume or molar basis), in a fraction or stream.

It should be understood that, in the specification, when a numerical range is defined by a lower limit and/or upper limit, the numerical range includes any subcombination thereof. For example, the range of "1 to 5" may include 1, 2, 3, 4, and 5, as well as any sub-combination therebetween.

It will be understood that, when an element or member is referred to as being "connected to" another element or member, it may be directly connected to the other element, or an intervening element or member may also be present therebetween, unless mentioned otherwise.

Similarly, it will also be understood that, when an element or member is referred to as "contacting" another element, it may directly contact the other element or an intervening element or member may be present therebetween.

It will also be understood that the terms "on" and "above" are used to describe positional relationship. Therefore, when a layer is referred to as being "on" or "above" another layer or element, it may be directly present on or above the other layer, or an intervening (intermediate) layer or element may also be interposed or present therebetween, unless mentioned otherwise. Similarly, expressions such as "under", "below", "beneath", and "between" may also be understood as relative position terms. In addition, the expression "sequentially" may also be understood as a relative position term.

It will be further understood that the term "comprises", when used in this specification, specifies the presence of elements, but does not preclude the presence or addition of one or more other elements and/or steps, unless mentioned otherwise.

According to one embodiment of the present disclosure, aromatic fractions as a feedstock are treated by a multistep process comprising selective hydrogenation and hydrogenation each performed in the presence of a heterogeneous catalyst (specifically, a supported catalyst in which a metal having hydrogenation activity is introduced into a support). As a result, it is possible to provide aromatic hydrocarbons (specifically, C8+ aromatic hydrocarbons) from which residual hydrogen, which acts as an obstacle to the operation of downstream processes (e.g., pump-based transfer, xylene separation, para-xylene recovery, and the like), is removed, while reducing the bromine index of aromatic hydrocarbon fractions.

### Selective hydrogenation

According to one embodiment of the present disclosure, an aromatic (specifically alkyl aromatic) hydrocarbon-containing fraction is first supplied as a feedstock. In this case, the feedstock may typically be a C8+ aromatic hydrocarbon-containing fraction and have a relatively high bromine index (BI) because it contains unsaturated hydrocarbons (specifically, olefins, diolefins, acetylene, and/or styrene (or derivatives thereof)).

In this regard, the alkyl aromatic hydrocarbon in the aromatic-containing hydrocarbon fraction may be, for example, an alkyl aromatic hydrocarbon having about 8 to 20 carbon atoms, specifically about 8 to 18 carbon atoms, and more specifically about 8 to 16 carbon atoms. The alkyl aromatic is a compound in which at least one alkyl is bonded to an aromatic ring and the alkyl may, for example, be a methyl, ethyl, propyl, or butyl group, or the like. The C8+ alkyl aromatic hydrocarbon may include, in addition to xylene, ethyltoluene, propylbenzene, tetramethylbenzene, ethyldimethylbenzene, diethylbenzene, methylpropylbenzene, ethylpropylbenzene, triethylbenzene, diisopropylbenzene, mixtures thereof, and the like.

In an exemplary embodiment, the feedstock may be a C8+ aromatic hydrocarbon fraction from which a C7- hydrocarbon fraction has been separated. The C8+ aromatic hydrocarbon-containing fraction is used because the C7- aromatic fraction discharged to the upper part of the separator (for example, the distillation column) for separating aromatic fractions into a C7- aromatic fraction and a C8+ aromatic fraction in a commercial process contains almost no bicyclic-aromatic hydrocarbon.

In addition, the aromatic hydrocarbon-containing fraction may contain unsaturated hydrocarbons that increase the bromine index (BI), for example, hydrocarbons having at least one double bond, and/or triple bond, such as monoolefins, diolefins, acetylene, and styrene (or derivatives thereof) and these unsaturated hydrocarbons are subject to selective hydrogenation. In addition, the content of unsaturated hydrocarbons in the aromatic-containing feedstock may be quantified by the bromine index. In this embodiment, the bromine index of the feedstock is, for example, at least about 30, specifically 50 to 30,000, specifically about 100 to about 20,000, particularly about 150 to about 10,000, and in certain embodiments, for example about 300 to about 3,000, specifically about 400 to about 2,000, and more specifically about 500 to 1,500.

According to an exemplary embodiment, the feedstock is an aromatic hydrocarbon-rich fraction, particularly a C8+ aromatic hydrocarbon-rich fraction, wherein the content of aromatics is, for example, at least about 50% by weight, specifically at least about 70% by weight, more specifically at least about 80% by weight, particularly at least about 90% by weight. In addition, the aromatic hydrocarbon-containing feedstock may contain saturated hydrocarbons, for example, about 50% by weight or less, specifically about 30% by weight or less, more specifically about 20% by weight or less, particularly specifically about 10% by weight or less.

According to exemplary embodiments, the aromatic hydrocarbon-containing feedstock may contain, in addition to monocyclic aromatic hydrocarbons, bicyclic aromatic hydrocarbons (e.g., at least one selected from naphthalene, indene, derivatives thereof, and the like). The content of such bicyclic-aromatic hydrocarbons may be, for example, at least about 0.3% by weight, specifically about 0.4 to about 20% by weight, more specifically about 0.5 to about 15% by weight, particularly specifically about 1 to about 10% by weight. However, the content of bicyclic aromatic hydrocarbons may vary widely depending on the source of the feedstock and is not limited to the range defined above. A lower amount of bicyclic-aromatic hydrocarbons may be present depending on the source.

According to exemplary embodiments, an aromatic hydrocarbon-containing feedstock may be derived by, for example, catalytic reforming of naphtha; thermal cracking reactions of naphtha, distillates or other hydrocarbons to produce light olefins and aromatic-rich fractions; or catalytic cracking or thermal cracking of heavy oil fractions to produce hydrocarbons having a boiling point similar to that of gasoline, and these sources may be used alone or in combination as a feedstock. In certain embodiments, the feedstock may be derived from catalytic reformate of naphtha.

According to an exemplary embodiment, the selective hydrogenation for removing unsaturated hydrocarbons from the aromatic-containing fraction is, for example, performed within the temperature range of room temperature to 300°C, specifically about 40 to 250°C, more specifically about 50 to 230°C. According to a specific embodiment, the selective hydrogenation is performed under a temperature condition of, for example, about 100 to 220°C, specifically about 120 to 215°C, more specifically about 130 to 210°C, particularly about 155 to 205°C. In this case, the selective hydrogenation may be performed as a liquid-phase reaction or a three-phase reaction (trickle bed) using an excess of hydrogen. However, a liquid-phase reaction capable of reducing investment costs and maintaining the amount of residual hydrogen as low as possible after the reaction may be more advantageous.

According to an exemplary embodiment, the amount of hydrogen fed during the selective hydrogenation is, for example, at least about 0.5 mol, specifically about 0.7 mol to 20 mol, more specifically about 1 to 10 mol, with respect to 1 mol of unsaturated hydrocarbon contained in the feedstock. When the amount of hydrogen supplied is excessively small or great, problems such as a low removal rate of unsaturated hydrocarbons or increased loss of aromatic hydrocarbons due to hydrogenation of aromatic rings may occur. However, the supply amount of hydrogen is not limited to the above range because it may be changed depending on the properties of the feedstock.

Meanwhile, according to exemplary embodiments, the pressure in the selective hydrogenation zone may be determined within the range of, for example, about 3 to 70 bar, specifically about 5 to 30 bar, and more specifically about 7 to 20 bar. When the pressure in the selective hydrogenation reaction zone is excessively low or high, problems such as a low removal rate of unsaturated hydrocarbons or increased loss of aromatic hydrocarbons due to hydrogenation of aromatic rings may occur. Therefore, the pressure in the selective hydrogenation zone is preferably controlled within the range defined above.

In addition, the liquid hourly space velocity (LHSV) of the aromatic-containing feedstock is, for example, about 0.3 to 30 hr⁻¹, specifically about 0.5 to 20 hr⁻¹, more specifically about 0.5 to 10 hr⁻¹.

Meanwhile, the catalyst (i.e., selective hydrogenation catalyst) used in the selective hydrogenation may be selected from catalysts having hydrogenation activity that are controlled to have substantially no hydrogenation selectivity for aromatic rings (mono- and/or bi- or multi-cyclic aromatic rings), but have high hydrogenation selectivity for unsaturated hydrocarbons.

According to an exemplary embodiment, the selective hydrogenation catalyst may comprise a support containing inorganic oxide, and an active metal including at least one selected from the group consisting of Ni, Pd, Pt, Ru, Rh, Re, Co, Mo, Co-Mo, Ni-Mo and Ni-W. In an exemplary embodiment, the content of the active metal (on an elemental basis) in the selective hydrogenation catalyst is, for example, about 0.5 to about 40% by weight, specifically about 2 to about 30% by weight, more specifically, about 4 to 25% by weight, based on the total weight of the catalyst. The range of the metal content is provided for illustration and may be changed depending on the type of active metal used, hydrogenation activity thereof, and the type of metal.

In the selective hydrogenation catalyst, the aspect of using a plurality of metals among the active metals exemplified above may be combined as exemplified below:
In the Co-Mo, the atomic ratio of cobalt to molybdenum may be, for example, 1 : about 0.5 to 10, specifically 1 : about 1 to 5, and more specifically 1 : about 1.5 to 3. In addition, in the Ni-Mo, the atomic ratio of nickel to molybdenum may be, for example, 1 : about 0.5 to 10, specifically 1 : about 1 to 5, and more specifically 1 : about 1.5 to 3. In addition, in the Ni-W, the atomic ratio of nickel to tungsten may be, for example, 1 : about 0.5 to 10, specifically 1 : about 1 to 5, more specifically 1 : about 1.5 to 3.

In one embodiment, the selective hydrogenation catalyst may be a reduced form, a sulfide form, or a combination thereof. As an example, a specific single active metal or a combination of two types of active metals may be both a reduced form and a sulfide form, and a different active metal or a combination of two types of active metals may be a reduced form. Typically, in the certain active metal or a combination thereof, a reduced form has a higher hydrogenation activity than the sulfide form and the form (reduced, sulfide or oxide form) of catalyst may be determined in consideration of the amount or partial pressure of hydrogen in contact with the catalyst.

According to another exemplary embodiment, the selective hydrogenation catalyst may be loaded into a single reactor in a stage loading manner. For example, a first selective hydrogenation catalyst layer and a second selective hydrogenation catalyst layer may be arranged in that order based on inflow direction of the feedstock. In this case, when the active metal in the first selective hydrogenation catalyst (or catalyst layer) is Re, Co, Mo, and/or Co-Mo, a reduced or sulfide catalyst layer is disposed, and when the first selective hydrogenation catalyst is Ni, Pd, Pt, Ru, Rh, Ni-Mo and/or Ni-W, a sulfide catalyst layer may be disposed. On the other hand, the second selective hydrogenation catalyst of the downstream process may have a configuration in which a catalyst layer in which a reduced form of Ni-Mo and/or Ni-W is supported is disposed.

In a modified example of the stage loading method described above, the selective hydrogenation may be performed by connecting a plurality of reactors corresponding to the first selective hydrogenation catalyst layer and the second selective hydrogenation catalyst layer in series.

The stage loading method or a modified example thereof is disclosed in US Patent Publication No. 2022-0073440 filed by the present applicant, which is incorporated herein by reference.

According to an exemplary embodiment, supports for the selective hydrogenation catalyst (or first and second selective hydrogenation catalysts) may be selected from inorganic oxides, specifically inorganic oxides having a great specific surface area. Such a support may, for example, include at least one selected from the group consisting of alumina, silica, silica-alumina, aluminum phosphate, zirconia, titania, bentonite, kaolin, clinoptilolite and montmorillonite. According to a specific embodiment, the inorganic oxide may be amorphous and, in particular, include at least one selected from the group consisting of alumina, silica and silica-alumina, particularly specifically, alumina.

According to an exemplary embodiment, the support may be cylindrical and may, for example, be about 0.5 to about 5 mm (specifically about 1 to 3 mm) in diameter, and about 3 to 20 mm (specifically about 5 to 15 mm) in dimension. Alternatively, the support may have a granule, pellet, tablet, or sphere shape, or the like, other than the cylinder shape. In this way, a molding method known in the art, extrusion, spray drying, pelletizing, oil dropping, or the like may be applied to produce a support having a specific shape, but this is provided for exemplary purposes.

According to exemplary embodiments, the support may have an apparent density of about 0.3 to 1.2 cc/g, specifically about 0.4 to 1.1 cc/g, and more specifically about 0.4 to 0.9 cc/g. In addition, the average pore diameter of the support may be, for example, about 3 to 1,000 nm, specifically about 5 to 800 nm, and more specifically about 7 to 600 nm. In addition, the specific surface area (BET) of the support may be, for example, about 10 to 1,000 m²/g, specifically about 30 to 800 m²/g, and more specifically about 50 to 600 m²/g. It may be understood that the numerical ranges of the physical properties are understood to be illustrative.

According to an exemplary embodiment, the method for supporting the active metal on the support in the hydrogenation catalyst is a method known in the art, for example, impregnation (e.g., incipient wetness impregnation, oversolution impregnation, or immersion), ion exchange, coprecipitation, or the like.

Typically, impregnation may be performed, for example, by filling the pores of the support with a mixture of a soluble metal precursor or compound (typically a watersoluble or solvent-soluble metal compound), specifically, a metal salt, with a liquid medium selected from water, acid aqueous solutions, and basic aqueous solutions, and the like.

In exemplary embodiments, useful metal precursors may generally be active metal salts, complexes and halides, and the like, and may be exemplified as follows.

For example, molybdenum precursors include at least one selected from molybdenum (II) acetate, ammonium (VI) molybdate, diammonium (III) dimolybdate, ammonium (VI) heptamolybdate, ammonium (VI) phosphomolybdate and similar sodium and potassium salts, molybdenum (III) bromide, molybdenum (III)-(V) chloride, molybdenum (VI) fluoride, molybdenum (VI) oxychloride, molybdenum (IV)-(VI) sulfide, molybdic acid and ammonium, sodium and potassium salts thereof, and molybdenum (II-VI) oxide, but are not necessarily limited thereto.

The cobalt precursor may include at least one selected from the group consisting of nitrates, sulfates, carbonates, acetates, alkoxides, and halides of cobalt. Specifically, the cobalt precursor, for example, includes at least one selected from cobalt nitrate, cobalt sulfate, cobalt acetate, cobalt carbonate, cobalt hydroxide, cobalt alkoxide, cobalt halide (e.g., cobalt chloride, cobalt bromide, and the like), hydrates thereof, and the like. More specifically, the cobalt precursor may be cobalt nitrate and/or a hydrate thereof (e.g., Co(NO₃)₂·6H₂O). The nickel precursor may, for example, include at least one selected from nickel nitrate, nickel sulfate, nickel phosphate, nickel halide, nickel carboxylate, nickel hydroxide, nickel carbonate, acetylacetonate nickel complexes, nickel acetate and hydrates thereof. More specifically, the nickel precursor may be nickel nitrate and/or a hydrate thereof (e.g., Ni(NO₃)₂·6H₂O). The tungsten precursor, for example, includes at least one selected from ammonium metatungstate, ammonium tungstate, sodium tungstate, tungstic acid, and tungsten chloride. The palladium precursor includes at least one selected from palladium acetate, palladium chloride, palladium nitrate, and palladium sulfate. The platinum precursor includes at least one selected from chloroplatinic acid, ammonium chloroplatinate, dinitrodiaminoplatinum, tetrachlorodiaminoplatinum, hexachlorodiaminoplatinum, dichlorodiaminoplatinum, platinum dichloride (II), platinum tetrachloride (IV), and the like. The ruthenium precursor may include at least one selected from ruthenium chloride, ruthenium nitrosyl nitrate, and chlorohexaaminoruthenium. The rhodium precursor may include at least one selected from rhodium chloride, rhodium acetate, rhodium nitrate, and rhodium sulfate. The rhenium precursor may include at least one selected from perrhenic acid, rhenium chloride, ammonium perrhenate, potassium perrhenate, and rhenium oxide.

According to exemplary embodiments, the concentration of the active metal in the impregnation solution may range, for example, from about 0.005 to about 5 M, specifically from about 0.01 to about 3 M, and more specifically from about 0.015 to about 2 M. Conditions for the impregnation are not particularly limited and the impregnation may be performed, for example, at about 1 to 100°C (specifically, about 25 to 60°C) for about 0.1 to 48 hours (about 0.5 to 12 hours), but these conditions may be understood as illustrative.

As described above, the support may be impregnated with the active metal and then dried. For example, the drying is performed under an oxygen-containing atmosphere (specifically, air) at a drying temperature, for example, of about 60 to about 200°C, specifically about 80 to about 150°C. In addition, the drying time may be determined within the range of, for example, about 0.5 to 15 hours, specifically about 1 to 12 hours. The drying enables the metal precursor to be more closely attached to the support.

Then, the dried catalyst is fired (or heat-treated) and the firing is performed under an oxygen-containing atmosphere (e.g., air) or an inert gas (e.g., nitrogen) atmosphere, specifically an oxygen-containing atmosphere. In addition, the firing temperature may be selected within the range of, for example, about 300 to 800°C, specifically about 400 to 650°C. In addition, the firing time may be controlled within the range of, for example, about 0.5 to 24 hours, specifically about 1 to 12 hours. When the firing is performed in an oxygen-containing atmosphere, active metals may be converted into oxides, for example molybdenum may be converted into MoO₃ and nickel may be converted into NiO.

### - Preparation of reduced-type catalyst

According to an exemplary embodiment, when the hydrogenation catalyst is a reduced type, reduction treatment may be performed to convert the active metal in the oxide-type catalyst into a completely reduced type and/or a partially reduced type.

In this regard, the reduction treatment may be performed using hydrogen alone or a dilution of hydrogen in an inert gas (e.g., N₂, He, Ar, or the like), for example, at a temperature of about 25 to 800°C, specifically about 200 to about 700°C, more specifically, of about 300 to about 550°C, and the reduction treatment time is not particularly limited and may be adjusted within the range of, for example, about 0.5 to 24 hours, specifically about 1 to 12 hours.

The reduction treatment enables active metals in the hydrogenation catalyst to be present in reduced forms. Illustratively, the metal contained in the catalyst may have an elemental form or a partially oxidized form (e.g., molybdenum (Mo) is present in a partially oxidized form of Mo⁴⁺ instead of Mo⁶⁺, which is the maximum oxidation state).

### - Preparation of sulfide-type catalyst

In order to control excessive hydrogenation activity that induces side reactions such as loss of aromaticity, or to impart a hydrogenation function (in particular, in the case of molybdenum), depending on the supported metal, the catalyst is optionally converted to a sulfide form rather than a reduced form.

According to an exemplary embodiment, the reduced catalyst may be sulfurized and the metal component in the catalyst may be converted into sulfide using a method known in the art. This sulfurization may be performed using either a gas-phase method (including contacting hydrogen sulfide or a mixture thereof with an inert gas) or a liquid-phase method (including contacting a sulfur compound-containing solution). According to certain embodiments, the reduced catalyst may be treated with a solution containing a sulfur compound.

According to an exemplary embodiment, the sulfur compound that can be used for sulfurization may include at least one selected from hydrogen sulfide, hydrogen disulfide, carbon disulfide, alkyl sulfide, and the like. In particular, the alkyl sulfide may, for example, be methyl sulfide, dimethyl sulfide, dimethyl disulfide, diethyl sulfide, and/or dibutyl sulfide. In addition, a hydrocarbon-based solvent, such as benzene, toluene, xylene, C9+ aromatics, hexane, or heptane, may be used as a solvent during the sulfurization. For example, the amount of the sulfur compound in the solution for sulfurization may be appropriately determined within an equivalent or higher than that required to sulfurize the metal in the catalyst. For example, when molybdenum is used as an active metal, a sulfur compound may be mixed in an equivalent or greater amount required to sulfurize molybdenum into MoS₃ (which may be finally converted into MoS₂) with a solution. Also, nickel may be converted into Ni₃S₂.

In an exemplary embodiment, the sulfurization may be performed at room temperature to about 500°C (specifically, about 100 to about 450°C) for about 0.5 to 100 hours (specifically, about 1 to about 48 hours).

Meanwhile, the bromine index of the product (i.e., the first aromatic hydrocarbon-containing product) decreases as the unsaturated hydrocarbons in the aromatic hydrocarbon-containing fraction are selectively removed through the selective hydrogenation. In an embodiment, the bromine index of the first aromatic hydrocarbon-containing product may be, for example, less than about 2,000, specifically less than about 1,000, and more specifically less than about 500. In this case, the first aromatic hydrocarbon-containing product has a bromine index reduced by at least about 20%, specifically about 30 to 99%, more specifically about 35 to 95%, and even more specifically about 40 to 90%, compared to the feedstock.

It may be preferable to hydrogenate and remove all unsaturated hydrocarbons during the selective hydrogenation described above. In this case, aromatic loss may also increase. Therefore, it may be advantageous to perform the reaction so as to have an appropriately balanced product distribution. In this case, the unsaturated carbons in the first aromatic hydrocarbon-containing product may be not completely removed and small amounts thereof may remain. In this regard, the content of unsaturated carbon in the first aromatic hydrocarbon-containing product is, for example, present in an amount of up to about 2% by weight, specifically up to about 1.5% by weight, more specifically about 0.01 to 1% by weight, particularly specifically about 0.02% by weight to 0.5% by weight.

Meanwhile, the hydrogen supplied during the selective hydrogenation may typically be used in excess of unsaturated hydrocarbons and there is a limit to removing all unsaturated hydrocarbons due to controlled hydrogenation activity, or the like. Therefore, the residual hydrogen is present in the first aromatic hydrocarbon-containing product. In this regard, the concentration of residual hydrogen in the first aromatic hydrocarbon-containing product may be, for example, up to about 200 wtppm, specifically up to about 180 wtppm, and more specifically up to about 150 wtppm. According to certain embodiments, the concentration of residual hydrogen in the first aromatic hydrocarbon-containing product may range, for example, from about 50 to 120 wtppm, specifically from about 60 to 110 wtppm, and more specifically from about 80 to 100 wtppm.

In addition, in an exemplary embodiment, it is preferable to minimize the loss of aromatic compounds during the selective hydrogenation. The total aromatic loss is, for example, less than about 0.45% by weight, specifically less than about 0.3% by weight, more specifically, less than about 0.1% by weight, particularly specifically, less than about 0.05% by weight.

### Hydrogenation

According to one embodiment of the present disclosure, additional hydrogenation may be performed to remove residual hydrogen present in the first aromatic hydrocarbon-containing product formed through selective hydrogenation of the aromatic fraction. This hydrogenation is different from the upstream selective hydrogenation in that it allows hydrogenation or saturation of an aromatic ring (specifically at least one double bond in an aromatic ring) having a particular structure in an aromatic hydrocarbon, based on the different hydrogenation reactivity between hydrocarbon structures, unlike the upstream selective hydrogenation, in which loss of the aromatic ring (monocyclic or multicyclic aromatic ring) due to hydrogenation is minimized. In addition, it is noteworthy that the hydrogenation is performed without supplying hydrogen from an external source because the hydrogenation reaction occurs using residual hydrogen.

According to the present embodiment, the residual hydrogen in the first aromatic hydrocarbon-containing product (i.e., selective-hydrogenated C8+ aromatic hydrocarbon fraction) can be effectively removed, based on the fact that the hydrogenation activity varies depending on the structure of the hydrocarbon during the hydrogenation of unsaturated hydrocarbons (specifically, olefins, etc.) using residual hydrogen in aromatic fractions.

Specifically, the hydrogenation activity of the compounds is evaluated in the order of olefins > bicyclic-aromatics (naphthalene → tetralin or indene → indane) > monocyclic aromatics. Residual hydrogen in aromatic fractions can be removed using olefins and/or bicyclic aromatic hydrocarbons with better hydrogenation reactivity, while maintaining the content of monocyclic aromatic hydrocarbons (i.e., minimizing the loss of monocyclic aromatic hydrocarbons), which are the target product, based on the difference in hydrogenation activity between hydrocarbon structures. In this regard, the term "bicyclic-aromatic" or "bicyclic-aromatic hydrocarbon" may refer to naphthalene and indene having two aromatic rings in the molecule.

According to this embodiment, in the additional hydrogenation, the bromine index resulting from the unsaturated hydrocarbon can be further lowered and the residual hydrogen present in the selective hydrogenation product can be removed by reacting (hydrogenating) the unsaturated hydrocarbon present in the first aromatic hydrocarbon-containing product with the residual hydrogen.

According to another exemplary embodiment, the first aromatic hydrocarbon-containing product further contains bicyclic-aromatic hydrocarbons in addition to unsaturated hydrocarbons. Specifically, when the feedstock (specifically, the C8+ aromatic hydrocarbon-containing fraction) contains a bicyclic aromatic in addition to the monocyclic aromatic, the aromatic ring is maintained as much as possible in the upstream selective hydrogenation (i.e., hydrogenation or saturation of the aromatic ring is suppressed as much as possible) and thus the bicyclic-aromatic hydrocarbon may still be present in the first aromatic hydrocarbon-containing product. In this case, the content of the bicyclic-aromatic hydrocarbon in the first aromatic hydrocarbon-containing product is, for example, at least about 0.3% by weight, specifically 0.4 to 15% by weight, more specifically about 0.5 to about 10% by weight, particularly specifically about 1 to about 5% by weight.

When the total amount of unsaturated hydrocarbons and/or bicyclic-aromatic hydrocarbons in the first aromatic hydrocarbon-containing product is less than the level required to remove residual hydrogen, prior to or during the hydrogenation reaction, for example, a bicyclic aromatic hydrocarbon may be further incorporated in or added to the reaction system, for example, the first aromatic hydrocarbon-containing product, so as to adjust the total amount to the range defined above. The amount of the bicyclic-aromatic hydrocarbon that is added is not necessarily limited because it may be changed depending on the content of residual hydrogen. As such, bicyclic-aromatic hydrocarbons are preferentially hydrogenated compared to monocyclic-aromatic hydrocarbons in terms of structural characteristics, so that loss of monocyclic-aromatic hydrocarbons such as C8 aromatics can be effectively suppressed.

In the present embodiment, hydrogenation of bicyclic-aromatic rings as well as unsaturated hydrocarbons is allowed in the hydrogenation and a trace amount of residual hydrogen needs to be minimized by the hydrogenation. For this reason, a catalyst having higher hydrogenation activity than the downstream selective hydrogenation may be used.

According to an exemplary embodiment, the hydrogenation catalyst may be prepared in a similar manner to the metal-supported catalyst used in the selective hydrogenation, the catalyst shape, and the like described above. In this regard, the active metal in the hydrogenation catalyst may comprise at least one selected from nickel and platinum group metals on the periodic table. When a platinum group metal is used as the active metal, the platinum group metal may comprise at least one selected from the group consisting of platinum, rhodium, and ruthenium. In addition, the active metal in the hydrogenation catalyst may be a reduced type, which is determined considering the fact that it exhibits a higher hydrogenation activity than a sulfide type. Particularly, among platinum group metals, palladium may not be preferred because a palladium catalyst may not provide sufficient hydrogenation activity to remove residual hydrogen compared to other platinum group metal catalysts.

In addition, the content of the active metal (on an element basis) in the hydrogenation catalyst is, for example, about 0.1 to 40% by weight, specifically about 0.2 to 30% by weight, more specifically about 0.5 to 25% by weight, particularly about 1 to 20% by weight, based on the total weight of the catalyst. The range of the metal content is provided for exemplary purposes and may be changed depending on the type of active metal used, hydrogenation activity thereof, and the type of metal.

Meanwhile, the support for supporting the active metal in the hydrogenation catalyst may be selected from the supports used in the selective hydrogenation catalyst and may be, specifically, alumina.

According to the present embodiment, most unsaturated hydrocarbons are removed to produce a selective hydrogenation product containing a trace amount of residual hydrogen in the upstream selective hydrogenation and the unsaturated hydrocarbons (and/or a bicyclic aromatic hydrocarbon) remaining in the selective hydrogenation product reacts with a trace amount of residual hydrogen without supplying hydrogen from an external source in the downstream hydrogenation. Therefore, the reaction conditions should be precisely controlled, compared to general hydrogenation of hydrocarbon fractions.

For example, the hydrogenation temperature may be controlled within the range of, for example, greater than about 55°C and less than about 255°C, specifically about 60°C to 230°C, and more specifically 70°C to 225°C. According to certain embodiments, the hydrogenation temperature may be determined within the range of about 80 to 185°C, specifically about 90 to 175°C, more specifically about 95 to 155°C, particularly specifically about 100 to 130°C.

When the hydrogenation temperature is excessively low, it may be difficult to remove substantially all of the residual hydrogen. Meanwhile, when the hydrogenation temperature is excessively high, the loss of monocyclic aromatic hydrocarbons and the bromine index (BI) of the product may increase. In particular, as the hydrogenation temperature increases, the difference in hydrogenation activity depending on the hydrocarbon structure may decrease. Furthermore, at an excessively high hydrogenation temperature, the conversion of bicyclic aromatic hydrocarbons, which are useful for removing residual hydrogen, does not increase, and rather, the loss of monocyclic aromatic hydrocarbons, which are target components, may increase. In consideration of this, it may be advantageous to properly adjust the reaction temperature within the range defined above.

Meanwhile, the pressure during the hydrogenation may increase the hydrogenation activity. As the reaction pressure increases, the loss of aromatics, particularly monocyclic aromatic hydrocarbons, may increase. In consideration of this, the reaction pressure may be adjusted within the range of, for example, about 3 to 60 bar, specifically about 4 to 30 bar, and more specifically about 5 to 15 bar.

According to an exemplary embodiment, the liquid hourly space velocity (LHSV) of the first aromatic hydrocarbon-containing product introduced into the hydrogenation reaction zone or reactor is, for example, adjusted within the range of about 0.3 to about 30 hr⁻¹, specifically about 0.5 to about 20 hr⁻¹, more specifically, about 0.5 to about 10 hr⁻¹.

As described above, the subsequent hydrogenation after selective hydrogenation enables formation of a second aromatic hydrocarbon-containing product in which the content of each of residual hydrogen and unsaturated hydrocarbons (additionally, bicyclic-aromatic hydrocarbons) is reduced. At this time, the amount of hydrogen (residual hydrogen) in the second aromatic hydrocarbon-containing product may be less than about 6 wtppm, specifically less than about 3 wtppm, and more specifically less than about 1 wtppm. In this regard, the reason for adjusting the amount of residual hydrogen to less than about 6 wtppm is as follows. The amount of residual hydrogen corresponding to the saturated solubility of hydrogen in aromatic hydrocarbons at 1 atm is 6 wtppm and when the amount of residual hydrogen is higher than this value, hydrogen bubbles may be formed, as described above, which may have an undesirable effect on the downstream equipment or processes. In this case, the saturated solubility of hydrogen may be measured with reference to the document of de Wet, W. J. J. S. Afr. Chem. Inst. 1964, 17, 9-13 and Satterfield, C. N. I. Chem. E. Symp. Ser. 1968, no. 28, 22-29, which is incorporated herein by reference.

In addition, the loss of monocyclic aromatic hydrocarbons in the hydrogenation process for the first aromatic hydrocarbon-containing product is, for example, less than about 0.15% by weight, specifically about 0.12% by weight or less, more specifically about 0.03% by weight or less, particularly specifically about 0.01% by weight or less.

According to an exemplary embodiment, the bromine index of the second aromatic hydrocarbon-containing product is reduced compared to the first aromatic hydrocarbon-containing product because unsaturated hydrocarbons and the like contained in the first aromatic hydrocarbon-containing product as a reactant are removed. For example, the bromine index of the xylene obtained by distillation of the second aromatic hydrocarbon-containing product is preferably low. In this regard, the level of the bromine index of the xylenes separated by distillation of the second aromatic hydrocarbon-containing product is, for example, preferably less than about 20, specifically less than about 15, and more specifically less than about 10, and this is advantageous for application to a downstream process using xylene having a bromine index of 20 or less as a raw material, particularly to a xylene separation and recovery process (e.g., a PAREX process).

### Integration with selective hydrogenation-hydrogenation and subsequent processes

According to one embodiment, in the purification process of treating an aromatic hydrocarbon-containing feedstock in the order of selective hydrogenation and hydrogenation, an exemplary aspect of removing residual hydrogen in the selective hydrogenated aromatic hydrocarbon-containing product by the downstream hydrogenation is shown in FIGS. 1A and 1B.

According to the illustrated embodiment, the overall process 100 or 200 may be performed in two aspects: a plurality of reactors connected in series (two reactors are used in FIG. 1A) and a stage loading method (two catalyst layers are sequentially stacked in FIG. 1B).

Referring to FIG. 1A, a first reactor A and a second reactor B correspond to a selective hydrogenation reactor and a hydrogenation reactor, respectively. An aromatic hydrocarbon-containing fraction 101 as a feedstock is combined with hydrogen 102 supplied from an external source and is introduced into the first reactor A as a stream 103 in which at least a part of the supplied hydrogen is dissolved in the feedstock. As described above, in the first reactor A, a reaction for selectively hydrogenating unsaturated hydrocarbons (olefins and the like) while maintaining an aromatic ring in the feedstock is performed and the selective hydrogenation product discharged therefrom, that is, a first aromatic hydrocarbon-containing product 104, is introduced into the second reactor B. In the second reactor B, unlike in the first reactor A, the residual hydrogen in the product 104 is removed by hydrogenation using unsaturated hydrocarbons and/or bicyclic-aromatic hydrocarbons while maintaining the monocyclic aromatic hydrocarbons in the product 104 as much as possible. As a result, the hydrogenation product discharged from the second reactor B, that is, the second aromatic hydrocarbon-containing product 105 can be obtained as a purified aromatic fraction.

In the embodiment shown in FIG. 1B, based on the flow direction of the feedstock in a single reactor, the first catalyst layer A' and the second catalyst layer B' are stacked in that order (i.e., stage loading). Similar to FIG. 1A, the aromatic-containing feedstock 201 combines with hydrogen 202 to form a stream 203 and the stream contacts multiple catalyst layers sequentially stacked within the reactor. In this case, the stream contacts the catalyst layer for selective hydrogenation A', and then contacts the catalyst layer for hydrogenation B' to produce a second aromatic hydrocarbon-containing product 205 having substantially no residual hydrogen.

A plurality of reactors connected in series as shown in FIG. 1A has an advantage of minimizing the loss of aromatics (in particular, monocyclic aromatic hydrocarbons such as C8 aromatics) using different operating conditions for respective reactors, but may have a disadvantage of increasing facility investment costs, compared to the aspect shown in FIG. 1B.

According to an exemplary embodiment, the second aromatic hydrocarbon-containing product is applied to the downstream process, specifically a xylene production process, particularly a para-xylene recovery process, and a purification process by two-stage hydrogenation may be integrated with a xylene production process.

As an example, the second aromatic hydrocarbon-containing product may be transferred to a xylene column and separated into a C8 aromatic hydrocarbon stream as an upstream and a C9+ aromatic hydrocarbon stream as a downstream. Then, the separated C8 aromatic hydrocarbon fraction is transferred to a para-xylene separation/recovery unit and para-xylene is then recovered therefrom. Representative examples of such para-xylene recovery technologies include UOP's Parex, IFP's Eluxyl, Toray's Aromax, and the like.

According to an exemplary embodiment, prior to separating the second aromatic hydrocarbon-containing product, treatment with a solid acid (solid acid catalyst) may optionally be performed. The solid acid is typically clay (natural and/or synthetic clays) and/or zeolites. Such a subsequent treatment enables the alkyl group of the C9+ aromatic hydrocarbon to be decomposed to partially increase the content of the C8- aromatic hydrocarbon. In this case, the clay may be used in the clay treatment of aromatic hydrocarbons known in the art and may, for example, include at least one selected from bentonite, montmorillonite, kaolin, and the like. Meanwhile, zeolite may, for example, include at least one selected from Zeolite Y. Zeolite X, ZSM-5, ZSM-11, ZSM-22, ZSM-23, ZSM-34, ZSM-35, ZSM-385, ZSM-48, ZSM-50, ZSM-57 and the like. In this case, the temperature for the solid acid treatment may be adjusted within the range to keep the second aromatic hydrocarbon-containing product in a liquid phase, for example, in the range of about 120 to about 350°C (specifically, about 160 to about 300°C). Further, the solid acid treatment pressure may be adjusted within the range of, for example, about 3 to 60 bar (specifically, about 5 to 30 bar). In addition, the space velocity may be determined within the range of, for example, about 0.2 to about 20 hr⁻¹ (about 0.5 to about 10 hr⁻¹). However, the treatment conditions may be understood as exemplary.

The C8 aromatic hydrocarbon fraction left after separation of para-xylene from the xylene recovery unit may contain mainly ortho-xylene and/or meta-xylene and may be transferred to the xylene isomerization unit to form a xylene mixture having an increased para-xylene content. The reaction in the xylene isomerization unit may be performed using known reaction conditions and catalysts and thus detailed descriptions thereof will be omitted. As such, the C8 isomerization product discharged through the xylene isomerization reaction is separated into a light fraction (e.g., a C7- hydrocarbon fraction (upstream) and a C8 aromatic hydrocarbon fraction (downstream) in a separation column. In this case, the C8 aromatic hydrocarbon fraction may be transferred to the xylene column.

### [Mode for Invention]

The present disclosure will be further clearly understood with reference to the following examples. However, the following examples are provided only for illustration of the present disclosure and thus should not be construed as limiting the scope of the present disclosure.

### EXAMPLES

The materials used in Examples and Comparative Examples are as follows.

ACS reagent-grade xylene, naphthalene and metal compounds were purchased from Sigma-Aldrich. The inorganic oxide was a commercially available product from Sigma-Aldrich. In addition, an aromatic-containing hydrocarbon fraction was obtained as a feedstock obtained through a commercial process and a C8+ aromatic fraction having a bromine index (BI) of 748 was used.

### Evaluation and comparison of xylene hydrogenation activity of respective catalysts

In Comparative Examples 1 to 11, catalysts were prepared to select catalysts suitable for each of the selective hydrogenation and the subsequent hydrogenation (removal of residual hydrogen) and hydrogenation activities thereof were evaluated.

### Comparative Example 1

### Xylene hydrogenation using Co reduced catalyst

Cobalt nitrate was dissolved in distilled water and the result was supported on an aluminum support by incipient wetness impregnation. Then, the impregnated support was maintained at room temperature for about 1 hour, dried at 150°C for 2 hours in an air atmosphere and fired at 500°C for 2 hours to prepare a Co/O supported catalyst having a Co content of 7% by weight.

40 cc of the CoO/alumina catalyst (catalyst size distribution: 20 to 40 mesh) was charged into a continuous fixed-bed reactor. Then, the atmosphere in the reactor was purged with nitrogen and the pressure was increased to 10 kgf/cm². Then, the nitrogen was replaced with hydrogen and reduction treatment was performed at an elevated temperature of 450°C 2 hours while feeding hydrogen at 500 cc/min.

Then, the temperature in the reactor was lowered to 100°C and then xylene hydrogenation was performed on hydrocarbon fraction (xylene : dimethylcyclohexane = 1 : 7) at a molar ratio of hydrogen/C8 aromatic of 6.0 and at a space velocity of 15 hr⁻¹. The results are shown in Table 1 below.

### Comparative Example 2

### Xylene hydrogenation using Mo sulfide catalyst

Ammonium heptamolybdate was dissolved in distilled water and the result was supported on an aluminum support by incipient wetness impregnation. Then, the impregnated support was maintained at room temperature for about 1 hour, dried at 150°C for 2 hours in an air atmosphere and fired at 500°C for 2 hours to prepare a Mo supported catalyst having a Mo content of 15% by weight.

40 cc of the prepared catalyst (catalyst size distribution: 20 to 40 mesh) was charged into a continuous fixed-bed reactor. Then, the atmosphere in the reactor was purged with nitrogen and the pressure was increased to 10 kgf/cm². Then, the nitrogen was replaced with hydrogen, hydrogen was fed at a flow rate of 500 cc/min while toluene mixed with 2% by weight of DMDS was continuously fed at 0.7 cc/min for 5 hours, and sulfurization was performed at an elevated temperature of 350°C for 6 hours. The reaction was performed under the same conditions as in Comparative Example 1 and the results are shown in Table 1 below.

### Comparative Example 3

### Xylene hydrogenation using CoMo sulfide catalyst

Ammonium heptamolybdate was dissolved in distilled water and the result was supported on an aluminum support by incipient wetness impregnation. Then, the impregnated support was maintained at room temperature for about 1 hour, dried at 150°C for 2 hours in an air atmosphere and fired at 500°C for 2 hours. Then, cobalt nitrate was dissolved in distilled water and nickel was further supported on a molybdenum-supported aluminum support by incipient wetness impregnation, followed by drying and firing under the same conditions as above to prepare a supported catalyst having a Co content of 3.5% by weight and a Mo content of 12% by weight.

Then, the reaction was performed under the same conditions as in Comparative Example 2 and the results are shown in Table 1 below.

### Comparative Example 4

### Xylene hydrogenation using NiMo sulfide catalyst

Ammonium heptamolybdate was dissolved in distilled water and the result was supported on an aluminum support by incipient wetness impregnation. Then, the impregnated support was maintained at room temperature for about 1 hour, dried at 150°C for 2 hours in an air atmosphere and fired at 500°C for 2 hours. Then, nickel nitrate was dissolved in distilled water and nickel was further supported on a molybdenum-supported aluminum support by incipient wetness impregnation, followed by drying and firing under the same conditions as above to prepare a supported catalyst having a Ni content of 3.5% by weight and a Mo content of 12% by weight.

The reaction was performed under the same conditions as in Comparative Example 2 and the results are shown in Table 1 below.

### Comparative Example 5

### Xylene hydrogenation using Mo reduced catalyst

Ammonium heptamolybdate was dissolved in distilled water and the result was supported on an aluminum support by incipient wetness impregnation. Then, the impregnated support was maintained at room temperature for about 1 hour, dried at 150°C for 2 hours in an air atmosphere and fired at 500°C for 2 hours to prepare a Mo-supported catalyst having a Mo content of 15% by weight.

The reaction was performed under the same conditions as in Comparative Example 1 and the results are shown in Table 1 below.

### Comparative Example 6

### Xylene hydrogenation using Ni reduced catalyst

Nickel nitrate was dissolved in distilled water and the result was supported on an aluminum support by incipient wetness impregnation. Then, the impregnated support was maintained at room temperature for about 1 hour, dried at 150°C for 2 hours in an air atmosphere and fired at 500°C for 2 hours to prepare a Ni-supported catalyst having a Ni content of 7% by weight. The reaction was performed under the same conditions as in Comparative Example 1 and the results are shown in Table 1 below.

### Comparative Example 7

### Xylene hydrogenation using Pd reduced catalyst

Palladium chloride was dissolved in distilled water and the result was supported on an aluminum support by incipient wetness impregnation. Then, the impregnated support was maintained at room temperature for about 1 hour, dried at 150°C for 2 hours in an air atmosphere and fired at 500°C for 2 hours to prepare a Pd-supported catalyst having a Pd content of 7% by weight. The reaction was performed under the same conditions as in Comparative Example 1 and the results are shown in Table 1 below.

### Comparative Example 8

### Xylene hydrogenation using Pt reduced catalyst

Chloroplatinic acid was dissolved in distilled water and the result was supported on an aluminum support by incipient wetness impregnation. Then, the impregnated support was maintained at room temperature for about 1 hour, dried at 150°C for 2 hours in an air atmosphere and fired at 500°C for 2 hours to prepare a Pt-supported catalyst having a Pt content of 7% by weight. The reaction was performed under the same conditions as in Comparative Example 1 and the results are shown in Table 1 below.

### Comparative Example 9

### Xylene hydrogenation using Ru reduced catalyst

Ruthenium chloride was dissolved in distilled water and the result was supported on an aluminum support by incipient wetness impregnation. Then, the impregnated support was maintained at room temperature for about 1 hour, dried at 150°C for 2 hours in an air atmosphere and fired at 500°C for 2 hours to prepare a Ru-supported catalyst having a Ru content of 7% by weight. The reaction was performed under the same conditions as in Comparative Example 1 and the results are shown in Table 1 below.

### Comparative Example 10

### Xylene hydrogenation using Rh reduced catalyst

Rhodium chloride as dissolved in distilled water and the result was supported on an aluminum support by incipient wetness impregnation. Then, the impregnated support was maintained at room temperature for about 1 hour, dried at 150°C for 2 hours in an air atmosphere and fired at 500°C for 2 hours to prepare a Rh-supported catalyst having a Rh content of 7% by weight. The reaction was performed in the same manner as in Comparative Example 1 and the results are shown in Table 1 below.

### Comparative Example 11

### Xylene hydrogenation using NiMo reduced catalyst

A catalyst prepared in the same manner as in Comparative Example 4 was reduced in the same manner as in Comparative Example 1 and the reaction was performed. The results are shown in Table 1 below.

**[Table 1]**

| Compara tive Example | 1 | 2 | 3 | 4 | 5 | 6 | 7 | 8 | 9 | 10 | 11 |
|---|---|---|---|---|---|---|---|---|---|---|---|
| catalys t | Co reduce d form | Mo sulfid e form | CoMo sulfide form | NiMo sulfid e form | Mo reduce d form | Ni reduce d form | Pd reduce d form | Pt reduce d form | Ru redu ced form | Rh reduc ed form | NiMo reduc ed form |
| Xylene convers ion (%) | 0.1 or less | 0.1 or less | 0.1 or less | 0.1 or less | 0.1 or less | 86.8 | 0.3 | 20.2 | 32.2 | 99.7 | 2.1 |

As can be seen from the table above, nickel and platinum-based catalysts (excluding palladium) have high hydrogenation activity and thus have high xylene hydrogenation conversion (loss), whereas Co reduced, Mo sulfide, CoMo sulfide, NiMo sulfide, Mo reduced, Pd reduced, and NiMo reduced catalysts have a xylene hydrogenation conversion of 2.1% or less. Among them, a catalyst having a xylene hydrogenation conversion of 0.1% by weight or less was used as a first catalyst for "selective hydrogenation". Meanwhile, the second catalyst for "hydrogenation" in the downstream stage should completely remove a small amount of hydrogen and thus a catalyst with a high hydrogenation activity having a xylene hydrogenation conversion of 20% or more was selected and used.

### Comparative Example 12

### Selective hydrogenation using Ni-Mo sulfide catalyst

Ammonium heptamolybdate was dissolved in distilled water and the result was supported on an aluminum support by incipient wetness impregnation. Then, the impregnated support was maintained at room temperature for about 1 hour, dried at 150°C for 2 hours in an air atmosphere and fired at 500°C for 2 hours. Then, nickel nitrate was dissolved in distilled water and nickel was further supported on a molybdenum-supported aluminum support by incipient wetness impregnation, followed by drying and firing under the same conditions as above to prepare a catalyst having a Ni content of 3.5% by weight and a Mo content of 12% by weight.

40 cc of the prepared catalyst (catalyst size distribution: 20 to 40 mesh) was charged into a continuous fixed-bed reactor. Then, the atmosphere in the reactor was purged with nitrogen and the pressure was increased to 10 kgf/cm². Then, the nitrogen was replaced with hydrogen, hydrogen was fed at a flow rate of 500 cc/min, while toluene mixed with 2% by weight of DMDS was continuously fed at 0.7 cc/min for 5 hours, and sulfurization was performed at an elevated temperature of 350°C for 6 hours.

Then, the temperature in the reactor was lowered to 185°C, the flow rate of H₂ was adjusted to 3.5 cc/min, and a C8+ aromatic fraction (BI: 748) was fed at 1.3 cc/min to perform hydrogenation. The reaction was performed for 2 days, and each obtained sample was subjected to BI measurement and gas chromatography. At this time, the bromine index of the product was 96 and the aromatics loss was 0.03% by weight.

In order to determine whether or not residual hydrogen is present after the reaction, discharge of residual hydrogen along with the product fraction through the connected glass line under normal temperature/pressure conditions was observed, and the volume of bubbles collected for a predetermined period of time and a fraction increase were measured. As a result, the content of residual hydrogen was about 120 wtppm.

### Comparative Example 13

### Selective hydrogenation using Ni-Mo sulfide catalyst

In order to suppress the presence of residual hydrogen in the selective hydrogenation product, the experiment was performed while reducing the amounts of the fed reactants compared to Comparative Example 12 to 1/2. Specifically, selective hydrogenation was performed under the same conditions as in Comparative Example 12, except that the flow rate of H₂ was changed to 1.75 cc/min and the flow rate of the C8+ aromatic fraction (BI: 748) was changed to 0.65 cc/min. At this time, the bromine index (BI) of the product was 94 and the aromatic loss was 0.03% by weight. In addition, it was found that unreacted residual hydrogen was discharged along with the product fraction through the connected glass line.

As such, when a catalyst having a relatively weak hydrogenation function was used, the olefin content could be reduced, while causing almost no aromatic loss by hydrogenation, but there was a limit to completely consuming the introduced hydrogen.

### Comparative Example 14

### Selective hydrogenation using Ni reduced catalyst

Nickel nitrate was dissolved in distilled water and the result was supported on an aluminum support by incipient wetness impregnation. Then, the impregnated support was maintained at room temperature for about 1 hour, dried at 150°C for 2 hours in an air atmosphere and fired at 500°C for 2 hours. At this time, the heating rate was adjusted to 3 °C/min. As a result, a NiO/alumina catalyst having a Ni content of 10% by weight was prepared.

40 cc of the prepared NiO/alumina catalyst (catalyst size distribution: 20 to 40 mesh) was charged into a continuous fixed-bed reactor. Then, the atmosphere in the reactor was purged with nitrogen and the pressure was increased to 10 kgf/cm². Then, the nitrogen was replaced with hydrogen and reduction treatment was performed at an elevated temperature of 450°C for 2 hours, while hydrogen was fed at a flow rate of 500 cc/min.

Then, the temperature in the reactor was lowered to 185°C, the flow rate of H₂ was adjusted to 3.5 cc/min, and a C8+ aromatic fraction (BI: 748) was fed at 1.3 cc/min to perform hydrogenation. The reaction was performed for 2 days, and each obtained sample was subjected to BI measurement and gas chromatography. At this time, the olefin conversion was 80% and the aromatics loss was 0.3 wt% (H₂/olefin = 2.7). In order to determine whether or not the residual hydrogen was present after the reaction, the product fraction was discharged through the connected glass line under normal temperature/pressure conditions. At this time, unreacted residual hydrogen was not discharged.

### Example 1

### Selective hydrogenation (Ni-Mo sulfide catalyst)-hydrogenation (Ni reduced catalyst) two-step process

First, the selective hydrogenation product prepared according to Comparative Example 12 was used as a reactant (bicyclic aromatic hydrocarbon content: 1.1% by weight) and was then subjected to hydrogenation. The catalyst for the hydrogenation was the nickel (reduced) catalyst used in Comparative Example 6 (40, catalyst size distribution: 20 to 40 mesh), the reaction pressure was 10 kgf/cm² (about 9.8 bar) and the C8+ aromatic fraction (BI: 96) was flowed at 1.3 cc/min to perform hydrogenation. At this time, in order to simulate the incorporation of residual hydrogen in the selective hydrogenation product (product of Comparative Example 12) and to conduct the experiment under the condition of a predetermined amount of residual hydrogen, hydrogen was injected to adjust the amount of residual hydrogen to 120 wtppm.

Hydrogenation was performed over 2 days, followed by sampling, measurement of bromine index (BI), and gas chromatography. In addition, whether or not residual hydrogen was present was determined and the results are shown in Table 2 below.

### Examples 2 to 4 and Comparative Examples 15 to 17

In order to determine the hydrogenation activity of each hydrocarbon structure depending on the reaction temperature, the experiment was performed in the same manner as in Example 1, and the results are shown in Table 2 below.

**[Table 2]**

| Item | Reaction temperature (°C) | BI | Conversion of bicyclic-aromatic (%) | C8-aromatic loss (wt%) | Presence of excess (residual) hydrogen |
|---|---|---|---|---|---|
| Comparative Example 15 | 25 | 46 | 5.5 | less than 0.005 | O |
| Comparative Example 16 | 55 | 35 | 10.4 | less than 0.005 | O |
| Example 1 | 105 | 5 | 31.0 | less than 0.005 | X |
| Example 2 | 155 | 3 | 30.1 | 0.008 | X |
| Example 3 | 185 | 12 | 28.5 | 0.013 | X |
| Example 4 | 205 | 15 | 26.4 | 0.023 | X |
| Comparative Example 17 | 255 | 45 | 8.3 | 0.150 | X |

As can be seen from the table above, residual hydrogen was still detected even after the hydrogenation of the residual hydrogen of 120 wtppm by use of Ni catalyst, when the reaction temperature was 55 °C or less (Comparative Examples 15 and 16). On the other hand, when the reaction temperature was 255 °C, the aromatic loss greatly increased and the bromine index (BI) in the hydrogenation product also increased. In particular, at a reaction temperature of 255°C (Comparative Example 17), the conversion of bicyclic aromatic did not increase, but rather the loss of C8 aromatics greatly increased.

It is considered that the result is due to the fact that the difference in hydrogenation activity depending on the hydrocarbon structure decreases as the reaction temperature increases.

### Comparative Example 18

### Selective hydrogenation (Ni-Mo sulfide catalyst)-hydrogenation (Pd reduced catalyst) two-step process

Experiments were performed in the same manner as in Example 1 using the Pd reduced catalyst prepared in Comparative Example 7 and the results are shown in Table 3 below.

### Example 5

### Selective hydrogenation (Ni-Mo sulfide catalyst)-hydrogenation (Rh reduced catalyst) two-step process

Experiments were performed in the same manner as in Example 1 using the Rh reduced catalyst prepared in Comparative Example 10 and the results are shown in Table 3 below.

### Example 6

### Selective hydrogenation (Ni-Mo sulfide catalyst)-hydrogenation (Ru reduced catalyst) two-step process

Experiments were performed in the same manner as in Example 1 using the Ru reduced catalyst prepared in Comparative Example 9 and the results are shown in Table 3 below.

### Example 7

### Selective hydrogenation (Ni-Mo sulfide catalyst)-hydrogenation (Pt reduced catalyst) two-step process

Experiments were performed in the same manner as in Example 1 using the Pt reduced catalyst prepared in Comparative Example 8 and the results are shown in Table 3 below.

**[Table 3]**

| Item | Reaction temperature (°C) | BI | Conversion of bicyclic-aromatic (%) | C8-aromatic loss (wt%) | Presence of excess (residual) hydrogen |
|---|---|---|---|---|---|
| Comparative Example 18 | 105 | 21 | 4.3 | less than 0.005 | O |
| Example 1 | 105 | 5 | 31.0 | less than 0.005 | X |
| Example 5 | 105 | 13 | 28.7 | 0.012 | X |
| Example 6 | 105 | 4 | 31.3 | less than 0.005 | X |
| Example 7 | 105 | 5 | 31.5 | less than 0.005 | X |

As can be seen from the table above, the catalysts with high hydrogenation activity (Ni, Rh, Ru, and Pt reduced catalysts) did not have residual hydrogen at the reaction temperature of 105°C, but the Pd reduced catalyst with relatively low hydrogenation activity failed to sufficiently remove the residual hydrogen.

### Long-term durability comparison

### Comparative Example 19

### Selective hydrogenation (Ni-Mo sulfide catalyst)-hydrogenation (Ni-Mo reduced catalyst) two-step process

The Ni-Mo oxide catalyst prepared in Comparative Example 4 was subjected to reduction pretreatment in the same manner as in Comparative Example 1 and the presence or absence of residual hydrogen was compared over the reaction time.

To compare the durability between the hydrogenation catalysts in the two-step process, the reactants were used in two-fold amounts, compared to Example 2. Specifically, the temperature in the reactor was adjusted to 155°C, the flow rate of H₂ was adjusted to 7.0 cc/min, and the C8+ aromatic fraction (BI: 748) was flowed at 2.6 cc/min to perform hydrogenation. The presence or absence of residual hydrogen and C8-aromatic loss (wt%) over reaction time are shown in Table 4 below.

### Example 8

### Selective hydrogenation (Ni-Mo sulfide catalyst)-hydrogenation (Ni reduced catalyst) two-step process

The Ni-Mo oxide catalyst prepared in Comparative Example 4 was subjected to reduction pretreatment in the same manner as in Comparative Example 1 and the results are shown in Table 4 below.

**[Table 4]**

| Item | After 2 days | | After 17 days | | After 60 days | |
|---|---|---|---|---|---|---|
| | Residual hydrogen | Aromatic loss | Residual hydrogen | Aromatic loss | Residual hydrogen | Aromatic loss |
| Comparative Example 19 | X | less than 0.005 | O | less than 0.005 | - | - |
| Example 8 | X | less than 0.005 | X | less than 0.005 | X | less than 0.005 |

As can be seen from the table above, in Example 8 in which the Ni reduced catalyst was used as the hydrogenation catalyst in the hydrogenation the two-step process, residual hydrogen was not observed even after 60 days, but in Comparative Example 19 in which the Ni-Mo reduced catalyst was used as the hydrogenation catalyst, residual hydrogen was observed after 17 days. In this case, frequent catalyst replacement is required.

### Analysis of effects of bicyclic aromatic hydrocarbons in selective hydrogenation products on removal of residual hydrogen during hydrogenation

### Comparative Example 20

A hydrogenation experiment was performed to remove residual hydrogen under the same conditions as in Example 2, except that a C8 aromatic hydrocarbon fraction having a bromine index (BI) of 65 and containing no bicyclic aromatic hydrocarbons was used, and the results are shown in Table 5 below.

**[Table 5]**

| Item | Reactant | | Hydrogenation product | | | |
|---|---|---|---|---|---|---|
| | BI | Bicyclic-aromatic (wt%) | BI | Bicyclic-aromatic (wt%) | C8 aromatic loss (wt%) | Presence of excess (residual) hydrogen |
| Example 2 | 96 | 1.1 | 3 | 0.77 | 0.008 | X |
| Comparative Example 20 | 65 | - | 5 | - | 0.190 | X |

As can be seen from the table above, in Example 2, the reactants contained bicyclic aromatic hydrocarbons with a higher hydrogenation activity than monocyclic aromatic hydrocarbons, so bicyclic-aromatic hydrocarbons were preferentially hydrogenated (0.33% by weight corresponding to about 30% of the bicyclic-aromatic hydrocarbons in the reactants is consumed).

As a result, the loss of C8 aromatic hydrocarbon, which is a monocyclic aromatic hydrocarbon, was much lower, compared to Comparative Example 20.

### Analysis of effect of amount of residual hydrogen in selective hydrogenation product on hydrogenation

### Comparative Example 21

The experiment was performed under the same conditions as in Example 3, except that hydrogen was injected such that the amount of residual hydrogen in the reactant (product of Comparative Example 1) was 210 wtppm. The results are shown in Table 6 below.

**[Table 6]**

| Item | Reactant | Product | | | |
|---|---|---|---|---|---|
| | Residual hydrogen (wtppm) | BI | Bicyclic-aromatic conversion | C8 aromatic loss (wt%) | Presence of excess (residual) hydrogen |
| Example 3 | 120 | 12 | 28.5 | 0.013 | X |
| Comparative Example 21 | 210 | 10 | 33.0 | 0.151 | X |

As can be seen from the table above, when the amount of residual hydrogen increased to 210 wtppm, the partial pressure of hydrogen in the reactant (i.e., the product of Comparative Example 1) increased, resulting in an increase in aromatic loss to 0.151% by weight.

The result is supposed to be due to the fact that, when the amount of residual hydrogen in the selective hydrogenation product is higher than a predetermined level, the difference in hydrogenation activity depending on the structure of the hydrocarbon also decreases due to the increased hydrogen partial pressure in the fraction. In particular, it is noteworthy that, compared to Example 3 (amount of residual hydrogen: 120 wtppm), Comparative Example 21 (amount of residual hydrogen: 210 wtppm) exhibited a greater increase in C8 aromatic loss than an increase in the conversion of bicyclic aromatic.

### Analysis of effects of reaction pressure on hydrogenation

### Examples 9 and 10

A hydrogenation was performed in the same manner as in Example 2, except that the reaction pressure was changed to 5 kgf/cm² (about 4.9 bar) and 30 kgf/cm² (about 29.4 bar). The results are shown in Table 7 below.

**[Table 7]**

| Item | Reaction pressure (kgf/cm²) | BI | Bicyclic-aromatic conversion (%) | C8 aromatic loss (wt%) | Presence of excess (residual) hydrogen |
|---|---|---|---|---|---|
| Example 2 | 10 | 3 | 30.1 | 0.008 | X |
| Example 9 | 5 | 17 | 29.0 | 0.006 | X |
| Example 10 | 30 | 25 | 27.3 | 0.025 | X |

As can be seen from the table above, as the reaction pressure increases, the loss of C8 aromatics (monocyclic aromatics) increases. However, although the reaction pressure was greatly increased to 30 kgf/cm², the loss of C8 aromatics was relatively low.

### Example 11

### Selective hydrogenation-hydrogenation using two reactors connected in series

Considering the experimental results according to Examples 1 to 10 and Comparative Examples 12 to 19, each of the two reactors (first reactor: selective hydrogenation; second reactor: hydrogenation) connected in series was filled with a catalyst and the C8+ aromatic fraction (BI: 748) was purified.

As in Comparative Example 12, the first reactor was filled with 40 cc of a catalyst and then sulfurization was performed, whereas, as in Comparative Example 14, the second reactor was filled with 40 cc of a catalyst and then reduction treatment was performed. The two reactors filled with the separately pretreated catalysts in series were connected to perform selective hydrogenation and hydrogenation under the same conditions as in Comparative Example 12. The product discharged from the second reactor was analyzed and the results are shown in Table 8 below.

At this time, in order to determine whether or not the second reactor connected in series removes excess (residual) hydrogen present in the fraction discharged from the first reactor, hydrogen and an aromatic fraction were injected only into the first reactor and the experiment was performed. The results are shown in Table 8 below.

### Example 12

### Selective hydrogenation-hydrogenation using reactors filled with two catalyst layers in stage loading manner

The same reactor was filled with the catalysts used in Comparative Example 12 and Comparative Example 14. At this time, the catalyst used in Comparative Example 12 (40 cc; selective hydrogenation catalyst) was disposed at the top of the reactor, whereas the catalyst used in Comparative Example 14 (40 cc; hydrogenation catalyst) was disposed at the bottom of the reactor. Selective hydrogenation and hydrogenation were performed under the same conditions as in Comparative Example 12. The results are shown in Table 8 below.

**[Table 8]**

| Item | Type of reactor | BI | C8 aromatic loss (wt%) | Presence of excess (residual) hydrogen |
|---|---|---|---|---|
| Example 11 | 2 Reactor | 14 | 0.04 | X |
| Example 12 | 2-Bed | 15 | 0.04 | X |

As can be seen from the table above, no difference was observed in bromine index reduction, residual hydrogen removal, C8 aromatic loss, and the like, depending on the type of reactor.

As described above, when selective hydrogenation is performed to remove unsaturated hydrocarbons contained in aromatic hydrocarbon-containing fractions, particularly C8+ aromatic hydrocarbon fractions, residual hydrogen in the product can be effectively removed while minimizing the loss of monocyclic aromatic hydrocarbons (in particular, C8 aromatics) in the selective hydrogenation product by controlling the reaction conditions (e.g., temperature and pressure) and, moreover, the content of bicyclic-aromatic hydrocarbons.

Although the preferred embodiments of the present disclosure have been disclosed for exemplary purposes, those skilled in the art will appreciate that various modifications, additions and substitutions are possible, without departing from the scope and spirit of the disclosure as disclosed in the accompanying claims.

## Claims

1. A process of purifying an aromatic hydrocarbon comprising:
a) performing selective hydrogenation of an aromatic hydrocarbon-containing feedstock having a bromine index of at least 30 in the presence of a selective hydrogenation catalyst while supplying hydrogen to form a first aromatic hydrocarbon-containing product having a reduced bromine index and unsaturated hydrocarbon content and containing residual hydrogen at up to 200 wtppm, and
b) performing hydrogenation of the first aromatic hydrocarbon-containing product using the residual hydrogen in the presence of a hydrogenation catalyst to form a second aromatic hydrocarbon-containing product containing residual hydrogen in a reduced amount, compared to the first aromatic hydrocarbon-containing product,
wherein the first aromatic hydrocarbon-containing product has a bromine index reduced by at least 20% compared to the aromatic hydrocarbon-containing feedstock, and contains (i) an unsaturated hydrocarbon and (ii) a bicyclic aromatic hydrocarbon.

2. The process according to claim 1, wherein the bicyclic-aromatic hydrocarbon is present in an amount of at least 0.3% by weight in the first aromatic hydrocarbon-containing product.

3. The process according to claim 2, wherein the hydrogenation of step (b) is performed by adding bicyclic-aromatic hydrocarbon to the first aromatic hydrocarbon-containing product, when the amount of the bicyclic-aromatic hydrocarbon in the first aromatic hydrocarbon-containing product is less than 0.3% by weight.

4. The process according to claim 1, wherein the bicyclic aromatic hydrocarbon comprises at least one selected from the group consisting of naphthalene, indene, and derivatives thereof.

5. The process according to claim 1, wherein a total aromatic loss in the first aromatic hydrocarbon-containing product is less than 0.45% by weight based on the weight of the aromatic hydrocarbon-containing feedstock, and a monocyclic aromatic loss in the second aromatic hydrocarbon-containing product is less than 0.15% by weight, based on the weight of the first aromatic hydrocarbon-containing product.

6. The process according to claim 1, wherein step a) and step b) are performed in a first reactor and a second reactor, respectively,
wherein the first reactor and the second reactor are connected in series.

7. The process according to claim 1, wherein step a) and step b) are each performed in the presence of a first catalyst layer and a second catalyst layer filled in a single reactor in a stage loading manner,
wherein the first catalyst layer and the second catalyst layer are arranged in the order based on the flow direction of the aromatic hydrocarbon-containing feedstock.

8. The process according to claim 1, wherein the hydrogenation of step b) is performed under reaction conditions controlled at a temperature higher than 55 °C and lower than about 255 °C and at a pressure of 3 to 60 bar.

9. The process according to claim 8, wherein the selective hydrogenation of step a) is performed at a temperature controlled in the range of 130 to 210 °C, and
the hydrogenation of step b) is performed at a temperature controlled in the range of 80 to 130 °C.

10. The process according to claim 1, wherein the hydrogenation catalyst of step b) comprises an active metal selected from at least one of nickel and platinum group metals and an inorganic oxide support.

11. The process according to claim 10, wherein the active metal in the hydrogenation catalyst in step b) comprises at least one selected from the group consisting of nickel, platinum, rhodium and ruthenium.

12. The process according to claim 10, wherein the hydrogenation catalyst is a reduced form.

13. The process according to claim 10, wherein an amount of the active metal in the hydrogenation catalyst in step b) is determined within the range of 0.1 to 40% by weight, on an elemental basis.

14. The process according to claim 1, wherein the aromatic hydrocarbon-containing feedstock comprises a C8+ aromatic hydrocarbon.

15. The process according to claim 1, further comprising:
c) separating the second aromatic hydrocarbon-containing product into a C9+ aromatic hydrocarbon fraction and a C8 aromatic hydrocarbon fraction; and
d) separating and recovering para-xylene from the separated C8 aromatic hydrocarbon fraction.

16. The process according to claim 15, further comprising treating the second aromatic hydrocarbon-containing product with a solid acid, prior to step c).

17. The process according to claim 15, further comprising:
e) isomerizing the residual C8 aromatic hydrocarbon fraction not recovered as para-xylene to form a C8 aromatic hydrocarbon fraction containing an increased content of para-xylene and then recycling to step (c).

18. The process according to claim 1, wherein the unsaturated hydrocarbon in the first aromatic hydrocarbon-containing product is present in an amount of 2% by weight or less.

19. The process according to claim 1, wherein the hydrogen in the second aromatic hydrocarbon-containing product is present in an amount of less than 6 wtppm.
